# EUROPEAN PATENT SPECIFICATION

(11) **EP 3 524 161 B1**
(45) Date of publication and mention of the grant of the patent: **09.11.2022**
(21) Application number: 18187745.7
(22) Date of filing: 07.08.2018
(51) Int. Cl.: A61B 8/00

(54) **ULTRASOUND IMAGING APPARATUS, METHOD OF CONTROLLING THE SAME, AND COMPUTER PROGRAM PRODUCT**
ULTRASCHALLBILDGEBUNGSVORRICHTUNG, VERFAHREN ZUR STEUERUNG DAVON UND COMPUTERPROGRAMM
APPAREIL D'IMAGERIE À ULTRASONS, SON PROCÉDÉ DE COMMANDE ET PRODUIT DE PROGRAMME INFORMATIQUE

(30) Priority: 13.02.2018 KR 20180018064
(43) Date of publication of application: 14.08.2019
(73) Proprietor: Samsung Medison Co., Ltd., Hongcheon-gun, Gangwon-do, 25108 (KR)
(72) Inventor: JIN, Gil-ju, Gyeonggi-do (KR); KANG, Young-seok, Gyeonggi-do (KR); AHN, Mi-jeoung, Gyeonggi-do (KR); KIM, Dae-hwan, Gyeonggi-do (KR)
(74) Representative: Hylarides, Paul Jacques

(56) References cited:
- JP-A- H0 678 920
- US-A- 5 501 221
- US-A1- 2003 073 894
- US-A1- 2015 359 516
- US-A1- 2016 345 936

## Description

### BACKGROUND

### 1. Field

The disclosure relates to ultrasound imaging apparatuses, methods of controlling the same, and a computer program product.

### 2. Description of Related Art

Ultrasound diagnosis apparatuses transmit ultrasound signals generated by transducers of a probe to an object and receive information of signals reflected from the object, thereby obtaining at least one image of an internal part (e.g., soft tissues or blood flow) of the object.

Document JP H06 78920 discloses an ultrasound imaging system, wherein gain compensation is set either via an operation panel or via a foot switch.

Document US2016 / 345936 discloses an ultrasound system comprising an ultrasound acquisition station and a remote evaluation station, wherein the user of the ultrasound acquisition station and the user of the ultrasound evaluation station can independently select the TGC for the ultrasound image to be displayed at the ultrasound acquisition station and the ultrasound evaluation station, respectively.

### SUMMARY

The invention is defined in the independent claims. Preferred embodiments are defined in the dependent claims.

Provided are methods and apparatuses that allow a client apparatus to control a time gain compensation (TGC) parameter of an ultrasound imaging apparatus.

Provided are methods and apparatuses that allow a client apparatus to adjust an analog TGC parameter and separately generate and provide an image to an ultrasound imaging apparatus and an image to the client apparatus.

Additional aspects will be set forth in part in the description which follows and, in part, will be apparent from the description, or may be learned by practice of the presented embodiments.

In accordance with an aspect of the disclosure, an ultrasound imaging apparatus according to claim 1 is provided.

The first TGC channel may include a first TGC compensator configured to compensate for an echo signal received from the probe by applying a first TGC set that is a set of gain values at reception depths.

The second TGC channel may include a second TGC compensator configured to compensate for an echo signal received from the probe by applying a second TGC set that is a set of gain values at reception depths. The first TGC set may be determined based on a control signal received from the ultrasound imaging apparatus, and the second TGC set may be determined based on a control signal received from the first client apparatus.

The one or more processors may be further configured to generate a live streaming signal from the second ultrasound image and transmit the live streaming signal to the first client apparatus via the communicator.

The ultrasound signal receiver may further include a third TGC channel, and the one or more processors may be further configured to change a TGC parameter of the third TGC channel, based on a control signal received from a second client apparatus, generate a third ultrasound image based on a third ultrasound signal produced via the third TGC channel, and transmit the third ultrasound image to the second client apparatus via the communicator.

The first client apparatus may be configured to receive a selection signal for selecting one of analog TGC and digital TGC, and the one or more processors may be further configured to change, based on reception of the selection signal for selecting the analog TGC by the first client apparatus, the second TGC parameter of the second TGC channel, based on the control signal from the first client apparatus.

The one or more processors may be further configured to perform, based on reception of the selection signal for selecting the digital TGC by the first client apparatus, the digital TGC on the second ultrasound image based on a TGC parameter included in the control signal from the first client apparatus and transmit the second ultrasound image that has undergone the digital TGC to the first client apparatus via the communicator.

The one or more processors may be further configured to provide via a display at least one or a combination of information about a first TGC parameter of the first TGC channel, information about the second TGC parameter of the second TGC channel, and information about which TGC parameter is used to generate a displayed ultrasound image.

The one or more processors may be further configured to change a first TGC parameter based on the second TGC parameter in response to one of a control signal received from the ultrasound imaging apparatus and the control signal received from the first client apparatus.

The ultrasound imaging apparatus may further include a storage storing information about the second TGC parameter according to a user of the first client apparatus, and the one or more processors may be further configured to set, based on information about the user of the first client apparatus, the second TGC parameter of the second TGC channel to be the second TGC parameter according to the user of the first client apparatus.

In accordance with another aspect of the disclosure, a method of controlling an ultrasound imaging apparatus according to claim 9 is provided.

In accordance with another aspect of the disclosure, a computer program product includes a non-transitory computer-readable recording medium having stored therein a computer program code for performing a method of controlling an ultrasound imaging apparatus.

### BRIEF DESCRIPTION OF THE DRAWINGS

The above and other aspects, features, and advantages of certain embodiments of the present disclosure will be more apparent from the following description taken in conjunction with the accompanying drawings, in which:
FIG. 1 is a block diagram of a configuration of an ultrasound diagnosis apparatus according to an embodiment;
FIGS. 2A through 2C respectively illustrate ultrasound diagnosis apparatuses according to embodiments;
FIG. 3 illustrates an ultrasound imaging apparatus and a client apparatus according to an embodiment;
FIG. 4 illustrates a structure of an ultrasound imaging apparatus and a first client apparatus, according to an embodiment;
FIG. 5 illustrates a structure of an ultrasound signal receiver according to an embodiment;
FIG. 6 is a flowchart of a method of controlling an ultrasound imaging apparatus, according to an embodiment;
FIG. 7 illustrates an ultrasound imaging apparatus and a plurality of client apparatuses according to an embodiment;
FIG. 8 illustrates a graphical user interface (GUI) view provided by a client apparatus, according to an embodiment;
FIG. 9 illustrates a GUI view for time gain compensation (TGC) control of an ultrasound imaging apparatus or a client apparatus, according to an embodiment;
FIG. 10 illustrates GUI views for TGC control of an ultrasound imaging apparatus and a client apparatus, according to an embodiment;
FIG. 11 illustrates a GUI view for TGC control of an ultrasound imaging apparatus or a client apparatus, according to an embodiment;
FIG. 12 illustrates a GUI view for TGC control of an ultrasound imaging apparatus or a client apparatus, according to an embodiment;
FIG. 13 illustrates a GUI view for TGC control of an ultrasound imaging apparatus or a client apparatus, according to an embodiment; and
FIG. 14 illustrates a GUI view for TGC control of an ultrasound imaging apparatus, according to an embodiment.

### DETAILED DESCRIPTION

Embodiments are described in greater detail below with reference to the accompanying drawings.

In the following description, the same drawing reference numerals are used for the same elements even in different drawings. The matters defined in the description, such as detailed construction and elements, are provided to assist in a comprehensive understanding of exemplary embodiments. Thus, it is apparent that exemplary embodiments can be carried out without those specifically defined matters. Also, well-known functions or constructions are not described in detail since they would obscure embodiments with unnecessary detail.

Terms such as "part" and "portion" used herein denote those that may be embodied by software or hardware. According to embodiments, a plurality of parts or portions may be embodied by a single unit or element, or a single part or portion may include a plurality of elements.

In embodiments, an image may include any medical image acquired by various medical imaging apparatuses such as a magnetic resonance imaging (MRI) apparatus, a computed tomography (CT) apparatus, an ultrasound imaging apparatus, or an X-ray apparatus.

Also, in the present specification, an "object", which is a thing to be imaged, may include a human, an animal, or a part thereof. For example, an object may include a part of a human, that is, an organ or a tissue, or a phantom.

Throughout the specification, an ultrasound image refers to an image of an object processed based on ultrasound signals transmitted to the object and reflected therefrom.

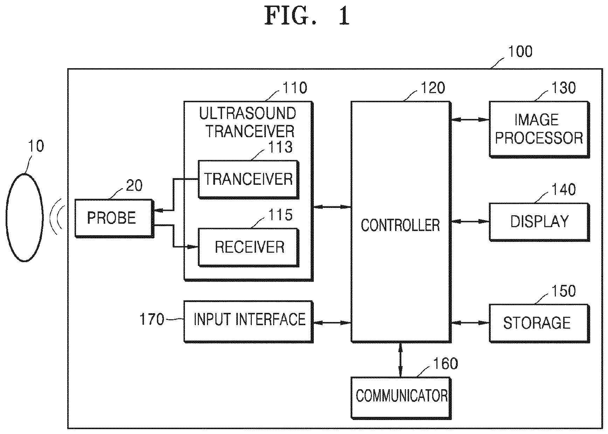

FIG. 1 is a block diagram illustrating a configuration of an ultrasound diagnosis apparatus 100, i.e., a diagnostic apparatus, according to an embodiment.

Referring to FIG. 1, the ultrasound diagnosis apparatus 100 may include a probe 20, an ultrasound transceiver 110, a controller 120, an image processor 130, one or more displays 140, a storage 150, e.g., a memory, a communicator 160, i.e., a communication device or an interface, and an input interface 170.

The ultrasound diagnosis apparatus 100 may be of a cart-type or a portable-type ultrasound diagnosis apparatus which is portable, moveable, mobile, or hand-held. Examples of the portable-type ultrasound diagnosis apparatus may include a smart phone, a laptop computer, a personal digital assistant (PDA), and a tablet personal computer (PC), each of which may include a probe and a software application, but embodiments are not limited thereto.

The probe 20 may include a plurality of transducers. The plurality of transducers may transmit ultrasound signals to an object 10 in response to transmitting signals received by the probe 20, from a transmitter 113. The plurality of transducers may receive ultrasound signals reflected from the object 10 to generate reception signals. In addition, the probe 20 and the ultrasound diagnosis apparatus 100 may be formed in one body (e.g., disposed in a single housing), or the probe 20 and the ultrasound diagnosis apparatus 100 may be formed separately (e.g., disposed separately in separate housings) but linked wirelessly or via wires. In addition, the ultrasound diagnosis apparatus 100 may include one or more probes 20 according to embodiments.

The controller 120 may control the transmitter 113 for the transmitter 113 to generate transmitting signals to be applied to each of the plurality of transducers based on a position and a focal point of the plurality of transducers included in the probe 20.

The controller 120 may control the receiver 115 to generate ultrasound data by converting reception signals received from the probe 20 from analogue to digital signals and summing the reception signals converted into digital form, based on a position and a focal point of the plurality of transducers.

The image processor 130 may generate an ultrasound image by using ultrasound data generated from the receiver 115.

The display 140 may display a generated ultrasound image and various pieces of information processed by the ultrasound diagnosis apparatus 100. The ultrasound diagnosis apparatus 100 may include two or more displays 140 according to the present embodiment. The display 140 may include a touch screen in combination with a touch panel.

The controller 120 may control the operations of the ultrasound diagnosis apparatus 100 and flow of signals between the internal elements of the ultrasound diagnosis apparatus 100. The controller 120 may include a memory for storing a program or data to perform functions of the ultrasound diagnosis apparatus 100 and a processor and/or a microprocessor (not shown) for processing the program or data. For example, the controller 120 may control the operation of the ultrasound diagnosis apparatus 100 by receiving a control signal from the input interface 170 or an external apparatus.

The ultrasound diagnosis apparatus 100 may include the communicator 160 and may be connected to external apparatuses, for example, servers, medical apparatuses, and portable devices such as smart phones, tablet personal computers (PCs), wearable devices, etc., via the communicator 160.

The communicator 160 may include at least one element capable of communicating with the external apparatuses. For example, the communicator 160 may include at least one among a short-range communication module, a wired communication module, and a wireless communication module.

The communicator 160 may receive a control signal and data from an external apparatus and transmit the received control signal to the controller 120 so that the controller 120 may control the ultrasound diagnosis apparatus 100 in response to the received control signal.

The controller 120 may transmit a control signal to the external apparatus via the communicator 160 so that the external apparatus may be controlled in response to the control signal of the controller 120.

For example, the external apparatus connected to the ultrasound diagnosis apparatus 100 may process the data of the external apparatus in response to the control signal of the controller 120 received via the communicator 160.

A program for controlling the ultrasound diagnosis apparatus 100 may be installed in the external apparatus. The program may include command languages to perform part of operation of the controller 120 or the entire operation of the controller 120.

The program may be pre-installed in the external apparatus or may be installed by a user of the external apparatus by downloading the program from a server that provides applications. The server that provides applications may include a recording medium where the program is stored.

The storage 150 may store various data or programs for driving and controlling the ultrasound diagnosis apparatus 100, input and/or output ultrasound data, ultrasound images, applications, etc.

The input interface 170 may receive a user's input to control the ultrasound diagnosis apparatus 100 and may include a keyboard, button, keypad, mouse, trackball, jog switch, knob, a touchpad, a touch screen, a microphone, a motion input means, a biometrics input means, etc. For example, the user's input may include inputs for manipulating buttons, keypads, mice, trackballs, jog switches, or knobs, inputs for touching a touchpad or a touch screen, a voice input, a motion input, and a bioinformation input, for example, iris recognition or fingerprint recognition, but an embodiment is not limited thereto.

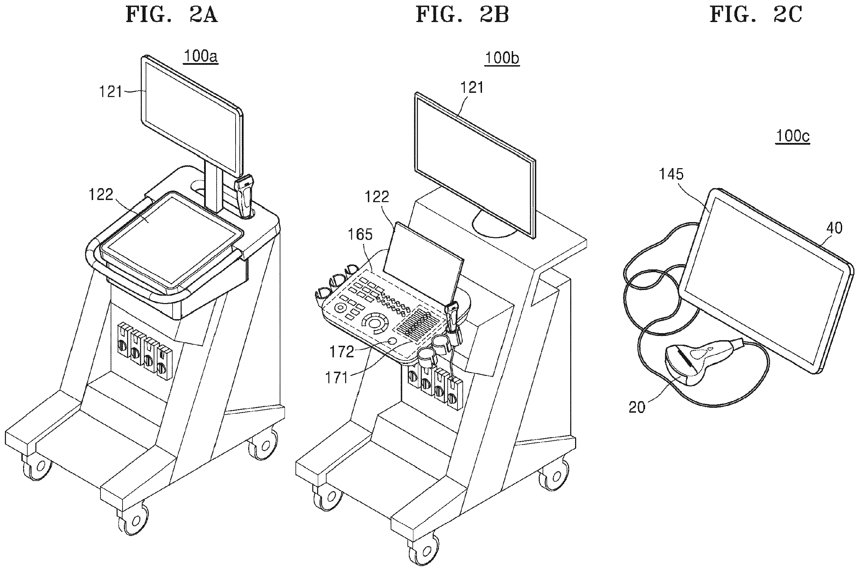

An example of the ultrasound diagnosis apparatus 100 according to the present embodiment is described below with reference to FIGS. 2A, 2B, and 2C.

FIGS. 2A, 2B, and 2C are diagrams illustrating ultrasound diagnosis apparatuses according to embodiments.

Referring to FIGS. 2A and 2B, an ultrasound diagnosis apparatus 100a or 100b may include a main display 121 and a sub-display 122. At least one among the main display 121 and the sub-display 122 may include a touch screen. The main display 121 and the sub-display 122 may display ultrasound images and/or various information processed by the ultrasound diagnosis apparatus 100a or 100b. The main display 121 and the sub-display 122 may provide graphical user interfaces (GUI), thereby receiving user's inputs of data to control the ultrasound diagnosis apparatus 100a or 100b. For example, the main display 121 may display an ultrasound image and the sub-display 122 may display a control panel to control display of the ultrasound image as a GUI. The sub-display 122 may receive an input of data to control the display of an image through the control panel displayed as a GUI. The ultrasound diagnosis apparatus 100a or 100b may control the display of the ultrasound image on the main display 121 by using the input control data.

Referring to FIG. 2B, the ultrasound diagnosis apparatus 100b may include a control panel 165. The control panel 165 may include buttons, trackballs, jog switches, or knobs, and may receive data to control the ultrasound diagnosis apparatus 100b from the user. For example, the control panel 165 may include a time gain compensation (TGC) button 171 and a freeze button 172. The TGC button 171 is to set a TGC value for each depth of an ultrasound image. Also, when an input of the freeze button 172 is detected during scanning an ultrasound image, the ultrasound diagnosis apparatus 100b may keep displaying a frame image at that time point.

The buttons, trackballs, jog switches, and knobs included in the control panel 165 may be provided as a GUI to the main display 121 or the sub-display 122.

Referring to FIG. 2C, the ultrasound diagnosis apparatus 100c may be implemented as a portable ultrasound diagnosis apparatus. An example of the portable ultrasound diagnosis apparatus may include, for example, smart phones including probes and applications, laptop computers, personal digital assistants (PDAs), or tablet PCs, but an embodiment is not limited thereto.

The ultrasound diagnosis apparatus 100c may include the probe 20 and a main body 40. The probe 20 may be connected to one side of the main body 40 by wire or wirelessly. The main body 40 may include a touch screen 145. The touch screen 145 may display an ultrasound image, various pieces of information processed by the ultrasound diagnosis apparatus 100c, and a GUI.

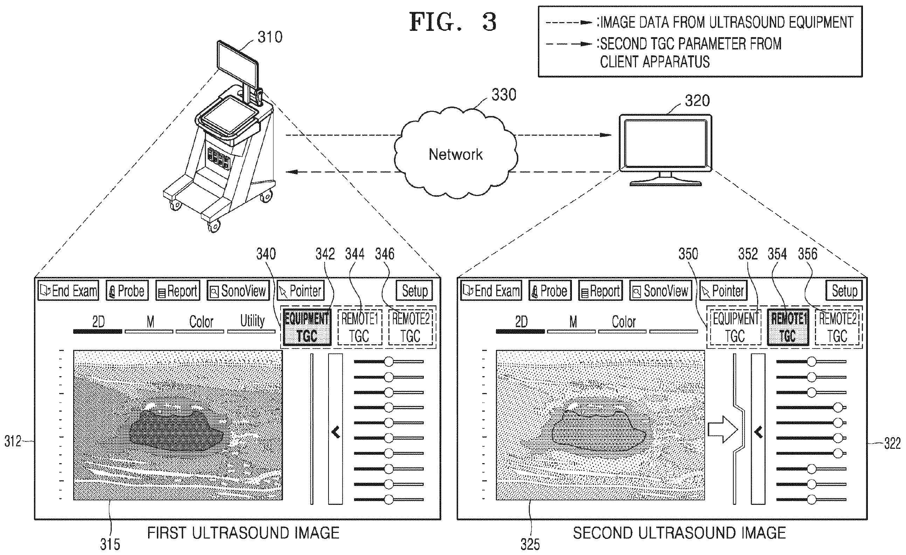

FIG. 3 illustrates an ultrasound imaging apparatus and a client apparatus according to an embodiment.

Referring to FIG. 3, an ultrasound imaging apparatus 310 transmits ultrasound signals generated by transducers of a probe to an affected area of a patient and receives information regarding ultrasound signals reflected from the patient to thereby reconstruct an image of a part or organ of the patient. When an ultrasound signal is transmitted to an affected area via a probe, the intensity of the ultrasound signal decreases with depth in the human body due to characteristics of ultrasound waves. TGC is used to compensate for the decreased intensity of the ultrasound signal. TGC is a method that uses preset values to vary the degree of compensation according to the time elapsed from the start of transmission of an ultrasound signal. The ultrasound signal, attenuation of which is compensated by the TGC, may then be converted into digital data by an analog-to-digital converter (ADC), and an image of an internal body part of a patient may be obtained based on the digital data.

The obtained image may be displayed on a display, be stored in the ultrasound imaging apparatus 310, or be transmitted to a server or a client apparatus 320. The client apparatus 320 may be used by a user, such as a medical doctor, to receive and view an ultrasound image.

The ultrasound imaging apparatus 310 has a live streaming function and may transmit an ultrasound image over a network 330 to provide the ultrasound image in real-time to the client apparatus 320 at a remote location. A live streaming technology may be used to provide various types of multimedia digital information such as video, audio, etc. to users who use external devices connected to a network such as the Internet. The users of the external devices may use live streaming functions to play back files such as video, audio, and the like in real-time on the Internet without downloading the files onto a hard disk drive.

Regarding the ultrasound imaging apparatus 310, a live streaming technology is used to transmit in real-time an ultrasound image being captured by the ultrasound imaging apparatus 310 to the client apparatus 320 at the remote location. By using the live streaming function provided by the ultrasound imaging apparatus 310, a user of the client apparatus 320 (e.g., a medical doctor) may view in real-time a video including ultrasound images of the internal body part of the patient that are being captured even when the user is not in an examination room where the patient is having an ultrasound scan. In detail, the user who uses the client apparatus 320 may quickly diagnose a disease of the patient based on the live streaming video including the ultrasound images provided via the client apparatus 320 and give feedback with respect to an imaged part or whether another scan of the patient is necessary.

During live streaming of an ultrasound image, when a TGC setting needs to be changed due to a failure of an operator of the ultrasound imaging apparatus 310 to properly use the ultrasound imaging apparatus, the user of the client apparatus 320 may induce a user of the ultrasound imaging apparatus 310 to change the TGC setting by using a voice call, etc.. However, when the user of the client apparatus 320 instructs the user of the ultrasound imaging apparatus 310 to change a TGC setting in this way, the intention of the user of the client apparatus 320 may not be properly delivered to the user of the ultrasound imaging apparatus 310, and changing the TGC setting indirectly via the user of the ultrasound imaging apparatus 310 may be inconvenient. Another inconvenience may arise from a difference between TGC settings respectively desired by the user of the ultrasound imaging apparatus 310 and the user of the client apparatus 320. According to the invention, the ultrasound imaging apparatus 310 allows the user of the 35 client apparatus 320 to directly change a second TGC setting of the ultrasound imaging apparatus 310. Furthermore, the ultrasound imaging apparatus 310 provides a first ultrasound image 315 generated by applying a first TGC set while the client apparatus 320 is provided with a second ultrasound image generated by applying the second TGC set.

In an embodiment, TGC settings are settings with respect to parameters related to a TGC function and include settings for gain values at reception depths. Furthermore, TGC parameters are parameters related to the TGC function and include gain values at the reception depths. A TGC set is a set of TGC parameters and includes a set of gain values at reception depths.

Embodiments may be applied to both TGC control and lateral gain compensation (LGC) control. LGC is a technique of varying the degree of compensation according to a lateral position. Analog LGC may be performed by applying, depending on a position of a transducer element, different gains to signals respectively detected by individual transducer elements or to sums of signals respectively detected by a predetermined number of transducer element arrays. Digital LGC may be performed by applying different gains according to a lateral position in an ultrasound image. The descriptions presented with respect to TGC may be also valid with respect to LGC. In an embodiment, the ultrasound imaging apparatus 310 may receive a control signal for setting an LGC parameter from the client apparatus 320 and perform an analog LGC operation based on LGC parameters received from the client apparatus 320.

According to an embodiment, the ultrasound imaging apparatus 310 may be connected to the client apparatus 320 via the network 330. The ultrasound imaging apparatus 310 may exchange data and a control signal with the client apparatus 320 via the network 330.

The network 330 may use various types of wired or wireless communication techniques. The ultrasound imaging apparatus 310 and the client apparatus 320 may be in the same room or be located in different rooms or buildings. Furthermore, the ultrasound imaging apparatus 310 may be connected to the client apparatus 320 directly or via a server.

According to the invention, the client apparatus 320 can adjust a TGC parameter of the ultrasound imaging apparatus 310. The client apparatus 320 may set the TGC parameter of the ultrasound imaging apparatus 310 via a UI for a TGC setting of the client apparatus 320. The ultrasound imaging apparatus 310 adjusts the TGC parameter based on a TGC setting control signal received from the client apparatus 320. According to an embodiment, the ultrasound imaging apparatus 310 may control an analog TGC parameter based on a control signal received from the client apparatus 320.

The ultrasound imaging apparatus 310 generates the first ultrasound image 315 by using a first TGC parameter set in the ultrasound imaging apparatus 310 and displays the first ultrasound image 315 via the ultrasound imaging apparatus 310. The ultrasound imaging apparatus 310 generates a second ultrasound image 325 by using a second TGC parameter and transmits the second ultrasound image 325 to the client apparatus 320.

According to an embodiment, the ultrasound imaging apparatus 310 may provide information indicating an apparatus that has a setting used to generate an image currently being displayed on a UI view 312. The UI view 312 of the ultrasound imaging apparatus 310 may include a first region 340 that provides information indicating an apparatus that has a setting used to generate an ultrasound image. In an embodiment, the first region 340 may include an icon 342 corresponding to the ultrasound imaging apparatus 310 and icons 344 and 346 respectively corresponding to at least one client apparatus 320. The ultrasound imaging apparatus 310 may display an ultrasound image generated based on a setting of an apparatus corresponding to the icon 342, 344, or 346 that is selected in response to a control signal for selecting one of the icons 342, 344, and 346. In an embodiment, when the user selects the icon 342, the ultrasound imaging apparatus 310 may display the first ultrasound image 315 based on its TGC setting. When the user selects the icon 344, the ultrasound imaging apparatus 310 may display a second ultrasound image based on a TGC setting of a first client apparatus. When the user selects the icon 346, the ultrasound imaging apparatus 310 may display a third ultrasound image based on a TGC setting of a second client apparatus.

According to an embodiment, the client apparatus 320 may provide information indicating an apparatus having a setting that is used to generate an image currently being displayed on a UI view 322. The UI view 322 of the client apparatus 320 may include a second region 350 that provides information indicating an apparatus having a setting that is used to generate an ultrasound image. In an embodiment, the second region 350 may include an icon 354 corresponding to the client apparatus 320, an icon 352 corresponding to the ultrasound imaging apparatus 310, and an icon 356 corresponding to another client apparatus. The client apparatus 320 may display an ultrasound image generated based on a setting of an apparatus corresponding to the icon 352, 354, or 356 that is selected in response to a control signal for selecting one of the icons 352, 354, and 356. In an embodiment, when the user selects the icon 352, the client apparatus 320 may display a first ultrasound image 315 based on a TGC setting of the ultrasound imaging apparatus 310. When the user selects the icon 354, the client apparatus 320 may display a second ultrasound image 325 based on its TGC setting. When the user selects the icon 356, the client apparatus 320 may display a third ultrasound image based on a TGC setting of the other client apparatus.

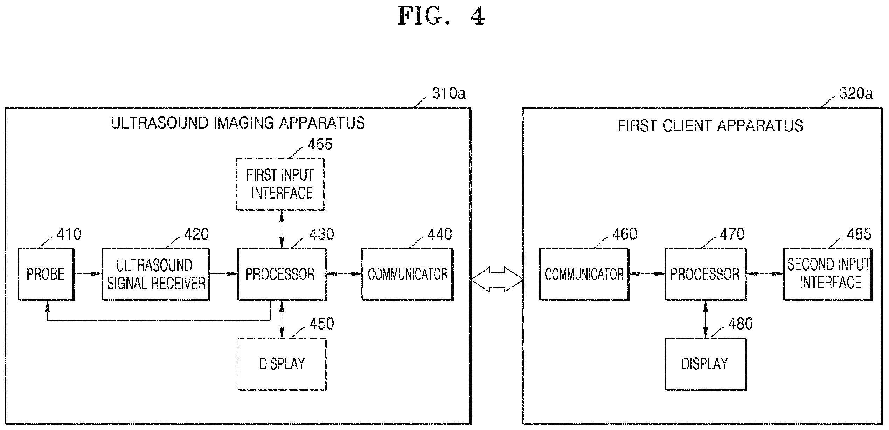

FIG. 4 illustrates a structure of an ultrasound imaging apparatus and a first client apparatus, according to an embodiment.

Referring to FIG. 4, an ultrasound imaging apparatus 310a may include a probe 410, an ultrasound signal receiver 420, a processor 430, and a communicator 440. According to an embodiment, the ultrasound imaging apparatus 310a may further include a display 450.

The probe 410 includes a plurality of transducers and transmits ultrasound signals to an object and detects echo signals reflected from the object. The probe 410 may correspond to the probe 20 described with reference to FIG. 1.

The ultrasound signal receiver 420 receives ultrasound signals generated by the probe 410 to perform operations such as compensation, filtering, and analog-to-digital conversion on the ultrasound signals, and converts the ultrasound signals into digital signals to output the digital signals to the processor 430. The ultrasound signals are electrical signals generated and output by the probe 410. The ultrasound signal receiver 420 may correspond to the receiver 115 described with reference to FIG. 1. According to an embodiment, the ultrasound signal receiver 420 may include a plurality of TGC channels. According to the invention, the ultrasound signal receiver 420 includes first and second TGC channels. The first TGC channel generates a first ultrasound signal by applying a first TGC parameter to a signal received from the probe 410. The second TGC channel generates a second ultrasound signal by applying a second TGC parameter to a signal received from the probe 410. The ultrasound signal receiver 420 outputs the first and second ultrasound signals to the processor 430.

The processor 430 controls all operations of the ultrasound imaging apparatus 310a. The processor 430 is implemented as one or more processors.

The processor 430 receives an ultrasound signal from the ultrasound signal receiver 420 to reconstruct an ultrasound image. According to an embodiment, the processor 430 may respectively generate first and second ultrasound images based on the first and second ultrasound signals received from the ultrasound signal receiver 420.

Furthermore, the processor 430 controls a TGC parameter of the first TGC channel based on a TGC control signal received from the ultrasound imaging apparatus 310a, and a TGC parameter of the second TGC channel based on a control signal received from a first client apparatus 320a.

The processor 430 sets a TGC parameter of the first TGC channel based on a control signal received from the ultrasound imaging apparatus 310a. In an embodiment, the ultrasound imaging apparatus 310a may include an input interface and set a TGC parameter of the first TGC channel based on a control signal received via the input interface. Furthermore, the processor 430 may provide a UI for setting a TGC parameter in the ultrasound imaging apparatus 310a and set the TGC parameter of the first TGC channel based on a control signal input via the UI. The processor 430 may perform analog TGC based on a TGC control signal received from the first client apparatus 320a by separately controlling TGC parameters of the first and second TGC channels.

The processor 430 may determine a gain value at each reception depth based on a TGC control signal received from the ultrasound imaging apparatus 310a and adjust a compensation value from a compensator, which corresponds to each reception depth for the first TGC channel, to control the first TGC parameter. Furthermore, the processor 430 may determine a gain value at each reception depth based on a TGC control signal received from the first client apparatus 320a and adjust a compensation value from a compensator, which corresponds to each reception depth for the second TGC channel, to control the second TGC parameter.

Furthermore, the processor 430 may provide a UI for TGC control in the ultrasound imaging apparatus 310a. The processor 430 may provide a GUI for TGC control via the display 450 of the ultrasound imaging apparatus 310a. According to an embodiment, the ultrasound imaging apparatus 310a is equipped with a touch screen, and the processor 430 may provide a GUI for TGC control via the touch screen and receive a TGC control signal via the touch screen.

According to an embodiment, the processor 430 may provide and control a part of or all of a GUI for TGC control of the first client apparatus 320a. In an embodiment, the processor 430 may provide data, a control signal, a program code, a link address, etc. for constructing the GUI for TGC control to the first client apparatus 320a.

The communicator 440 communicates with the first client apparatus 320a. The communicator 440 may transmit or receive control signals and data to or from the first client apparatus 320a. The communicator 440 transmits a second ultrasound image to the first client apparatus 320a and receives a TGC control signal from the first client apparatus 320a. Furthermore, the communicator 440 may transmit a live streaming image to the first client apparatus 320a as an ultrasound image.

The communicator 440 may perform local area communication and use communication technologies such as Bluetooth, Bluetooth low energy (BLE), near field communication (NFC), wireless LAN (WLAN), Wi-Fi, ZigBee, infrared data association (IrDA) communication, Wi-Fi Direct (WFD), ultra wideband (UWB), and Ant+. As another example, the communicator 440 may use a mobile communication method to transmit or receive wireless signals to or from at least one of a base station, an external terminal, and a server on a mobile communication network.

According to an embodiment, the ultrasound imaging apparatus 310a may further include a first input interface 455. The first input interface 455 may receive a control signal from the outside (e.g., a user). For example, the first input interface 455 may include at least one or a combination of a touch screen, a touch pad, a trackball, a knob, a dial, a keyboard, and a mouse. The first input interface 455 may correspond to the input interface described with reference to FIG. 1. The first input interface 455 may receive a TGC control signal for selecting a TGC parameter. The TGC control signal may be received in various forms, such as a control signal for selecting a TGC value according to a reception depth, a control signal for drawing a TGC curve, etc. An LGC control signal may be received in various forms, such as a control signal for selecting an LGC value according to a lateral position, a control signal for drawing an LGC curve, etc.

The first client apparatus 320a includes a communicator 460, a processor 470, and a display 480. For example, the first client apparatus 320a may be formed as a smartphone, a tablet PC, a communication terminal, a PC, a kiosk, or the like.

The communicator 460 communicates with the ultrasound imaging apparatus 310a. The communicator 460 may receive a second ultrasound image from the ultrasound imaging apparatus 310a and transmit a control signal for setting a second TGC parameter to the ultrasound imaging apparatus 310a. Furthermore, the communicator 460 may receive a live streaming signal with respect to the second ultrasound image from the ultrasound imaging apparatus 310a. The communicator 460 may communicate with the ultrasound imaging apparatus 310a wirelessly or by wire by using a communication method such as local area communication, mobile communication, etc.

The processor 470 controls all operations of the first client apparatus 320a. The processor 470 may be implemented as one or more processors. The processor 470 may provide the second ultrasound image and a UI for TGC control via the display 480 of the client apparatus 320a. For example, the display 480 may be formed as a touch screen, and the processor 470 may receive a user input for setting a second TGC parameter based on a touch input.

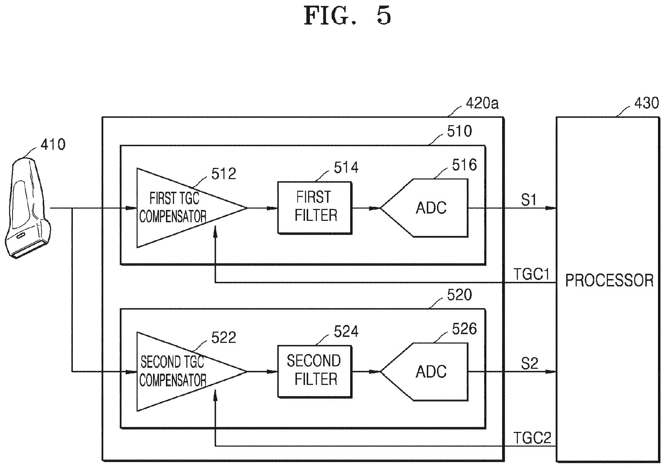

FIG. 5 illustrates a structure of an ultrasound signal receiver according to the invention.

The ultrasound signal receiver 420a includes a plurality of TGC channels such as a first TGC channel 510 and a second TGC channel 520. Each of the plurality of TGC channels performs TGC on an ultrasound signal received from the probe 410. In detail, a TGC channel may perform TGC by applying a different gain value to an ultrasound signal at each reception depth via a compensator. The TGC channel may perform analog TGC by compensating for an analog ultrasound signal. The ultrasound signal receiver 420a has the plurality of TGC channels that each perform analog TGC based on a control signal received from different apparatuses (320a of FIG. 4) to thereby obtain ultrasound images that are different from a first ultrasound image for the ultrasound imaging apparatus (310a of FIG. 4).

The first TGC channel 510 may include a first TGC compensator 512, a first filter 514, and a first ADC 516. The first TGC compensator 512 may perform an analog TGC operation by applying different gain values to an ultrasound signal received from the probe 410 according to a reception depth to thereby compensate for attenuation of the ultrasound signal. The first TGC compensator 512 may receive a first TGC parameter TGC1 from a processor 430 to perform the analog TGC operation based on the first TGC parameterTGC1. The first filter 514 performs an operation such as noise filtering with respect to a first ultrasound signal output from the first TGC compensator 512. The first ADC 516 then performs analog-to-digital conversion with respect to a first ultrasound signal output from the first filter 514. The first TGC channel 510 outputs first ultrasound signal S1 to the processor 430.

The second TGC channel 520 may include a second TGC compensator 522, a second filter 524, and a second ADC 526. The second TGC compensator 522 performs an analog TGC operation by applying different gain values to an ultrasound signal received from the probe 410 according to a reception depth to thereby compensate for attenuation of the ultrasound signal. The second TGC compensator 522 may receive a second TGC parameter TGC2 from the processor 430 to perform the analog TGC operation based on the second TGC parameterTGC2. The second filter 524 performs an operation such as noise filtering with respect to a second ultrasound signal output from the second TGC compensator 522. The second ADC 526 then performs analog-to-digital conversion with respect to a second ultrasound signal output from the second filter 524. The second TGC channel 520 outputs second ultrasound signal S2_to the processor 430.

The ultrasound signal receiver 420a may include three or more TGC channels according to an embodiment, and each TGC channel may correspond to a different apparatus.

When the present embodiment is applied to LGC, first and second LGC channels may be provided in the ultrasound signal receiver (420 of FIG. 4). The first LGC channel may perform analog LGC based on a first LGC parameter set in the ultrasound imaging apparatus (310 of FIG. 3), and the second LGC channel performs analog LGC based on a second LGC parameter received from the client apparatus (320 of FIG. 3). Furthermore, the processor 430 may generate a first ultrasound image based on a first ultrasound signal that passed through the first TGC channel 510 and the first LGC channel, and a second ultrasound image based on a second ultrasound signal (that passed through the second TGC channel 520 and the second LGC channel.

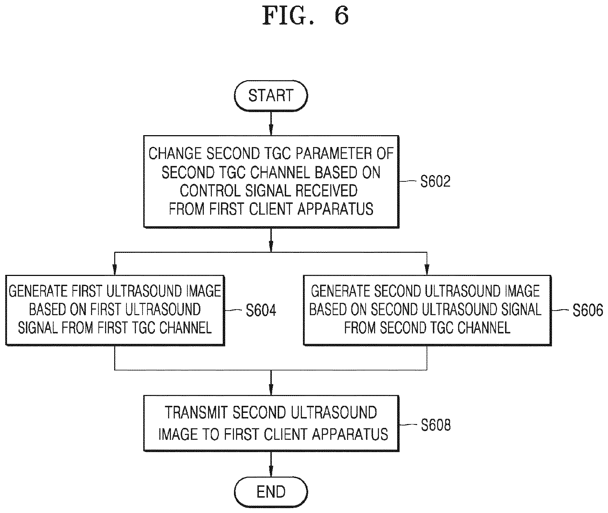

FIG. 6 is a flowchart of a method of controlling an ultrasound imaging apparatus, according to the invention.

Operations of the method of FIG. 6 are performed by an ultrasound imaging apparatus including a plurality of TGC channels and having a communication function that enables communication with a client apparatus. The specification mainly describes an embodiment in which an ultrasound imaging apparatus 310 (310 is hereinafter used as a reference numeral collectively denoting ultrasound imaging apparatuses disclosed herein) according to embodiments performs a method of controlling the ultrasound imaging apparatus. Thus, embodiments described with respect to the ultrasound imaging apparatus 310 may be applied to a method of controlling the ultrasound imaging apparatus 310, and embodiments described with respect to the method may be applied to the embodiments described with respect to the ultrasound imaging apparatus 310. A method of controlling the ultrasound imaging apparatus 310 according to embodiments is performed by the ultrasound imaging apparatus 310 described herein, but embodiments are not limited thereto. The method may be performed by various types of ultrasound imaging apparatuses.

The ultrasound imaging apparatus 310 receives a control signal for setting a second TGC parameter from the first client apparatus (320a of FIG. 4) and changes a TGC parameter of a second TGC channel based on the control signal received from the first client apparatus 320a (S602).

The ultrasound imaging apparatus 300 produces a first ultrasound signal via a first TGC channel by performing analog TGC based on a first TGC parameter set in the ultrasound imaging apparatus 310 and generates a first ultrasound image based on the first ultrasound signal (S604). The ultrasound imaging apparatus 310 may display the first ultrasound image on the display (450 of FIG. 4) of the ultrasound imaging apparatus 310.

The ultrasound imaging apparatus 310 produces a second ultrasound signal via a second TGC channel by performing analog TGC based on a second TGC parameter received from the first client apparatus 320a and generates a second ultrasound image based on the second ultrasound signal (S606).

The ultrasound imaging apparatus 310 transmits the second ultrasound image to the first client apparatus 320a (S608).

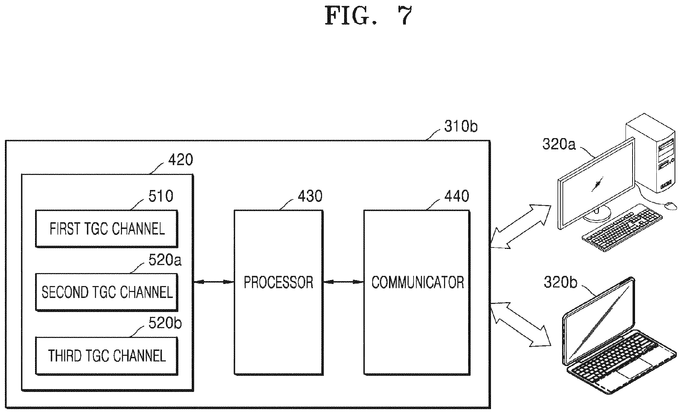

FIG. 7 illustrates an ultrasound imaging apparatus and a plurality of client apparatuses according to an embodiment.

According to an embodiment, an ultrasound imaging apparatus 310b may respectively transmit ultrasound images to and receive TGC parameters from a plurality of client apparatuses 320a and 320b. An ultrasound signal receiver 420 of the ultrasound imaging apparatus 310b may include three or more TGC channels. The ultrasound signal receiver 420 may include a plurality of TGC channels, i.e., first through third TGC channels 510, 520a, and 520b, and support a function of remotely performing analog TGC with respect to a number of client apparatuses. The number of client apparatus is one (1) less than the number of TGC channels. In an embodiment, when the ultrasound signal receiver 420 includes four (4) TGC channels that provide remote analog TGC functions with respect to three (3) client apparatuses. According to the embodiment of FIG. 7, the first TGC channel 510 may perform analog TGC based on a TGC parameter received from the ultrasound imaging apparatus 310b, and the second and third TGC channels 520a and 520b may respectively perform analog TGCs based on TGC parameters received from the plurality of client apparatuses 320a and 320b.

A processor 430 may respectively assign the second and third TGC channels 520a and 520b to the plurality of client apparatuses 320a and 320b and transmit the TGC parameters respectively received from the plurality of client apparatuses 320a and 320b to the assigned second and third TGC channels 520a and 520b. Furthermore, when the client apparatus 320a or 320b is connected to the ultrasound imaging apparatus 310b, the processor 430 may assign a corresponding TGC channel 520a or 520b to the connected client apparatus 320a or 320b. On the other hand, when the client apparatus 320a or 320b is disconnected from the ultrasound imaging apparatus 310b, the processor 430 may cancel assignment of a corresponding TGC channel 520a or 520b to the disconnected client apparatus 320a or 320b. The processor 430 may respectively generate ultrasound images based on ultrasound signals produced via the second and third TGC channels 520a and 520b and transmit the ultrasound images to the plurality of client apparatuses 320a and 320b to which the second and third TGC channels 520a and 520b are respectively assigned. For example, the processor 430 may transmit to the client apparatus 320a a second ultrasound image generated based on a second ultrasound signal produced via the second TGC channel 520a. The processor 430 may also transmit to the client apparatus 320b a third ultrasound image generated based on a third ultrasound signal produced via the third TGC channel 520b.

A communicator 440 may communicate with the plurality of client apparatuses 320a and 320b and respectively transmit corresponding ultrasound images thereto. Furthermore, the communicator 440 may respectively receive TGC parameters from the plurality of client apparatuses 320a and 320b.

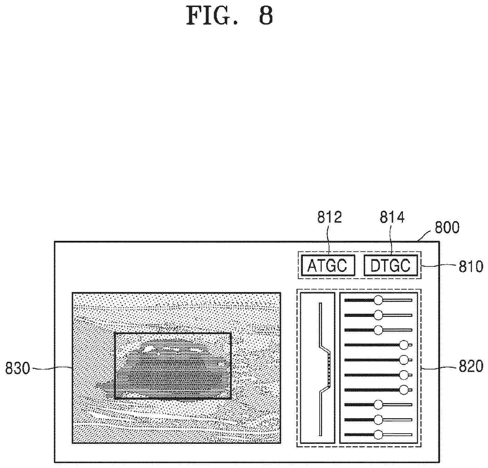

FIG. 8 illustrates a GUI view 800 provided by the client apparatus (320 of FIG. 3), according to an embodiment.

According to an embodiment, the client apparatus 320 may receive a user input of selecting analog TGC or digital TGC to perform a TGC operation selected by the user. For example, the client apparatus 320 may receive a user's selection by providing a TGC method selection menu 810 via a display.

The GUI view 800 may include the TGC method selection menu 810, a TGC parameter input portion 820, and a second ultrasound image 830. The TGC method selection menu 810 may include an analog TGC selection icon 812 and a digital TGC selection icon 814 and be provided to receive a user input of selecting a TGC method. The TGC parameter input portion 820 may be used to receive a user input of setting a second TGC parameter. The second ultrasound image may be provided as a streaming image via the display.

When analog TGC is selected in the client apparatus 320, the client apparatus 320 transmits the set second TGC parameter to the ultrasound imaging apparatus 310. According to an embodiment, the client apparatus 320 may transmit information about the selected analog TGC together during transmission of the second TGC parameter. According to an embodiment, the ultrasound imaging apparatus 310 may assign a TGC channel to the client apparatus 320 based on selection of the analog TGC in the client apparatus 320.

According to an embodiment, which is not part of the invention, when digital TGC is selected in the client apparatus 320, the client apparatus 320 may perform the digital TGC with respect to the second ultrasound image 830 based on the second TGC parameter.

When digital TGC is selected in the client apparatus 320, the client apparatus 320 transmits the second TGC parameter and information indicating selection of the digital TGC to the ultrasound imaging apparatus 310, and the ultrasound imaging apparatus 310 may perform the digital TGC on the second ultrasound image based on the second TGC parameter and then transmit the second ultrasound image that has undergone the digital TGC to the client apparatus 320.

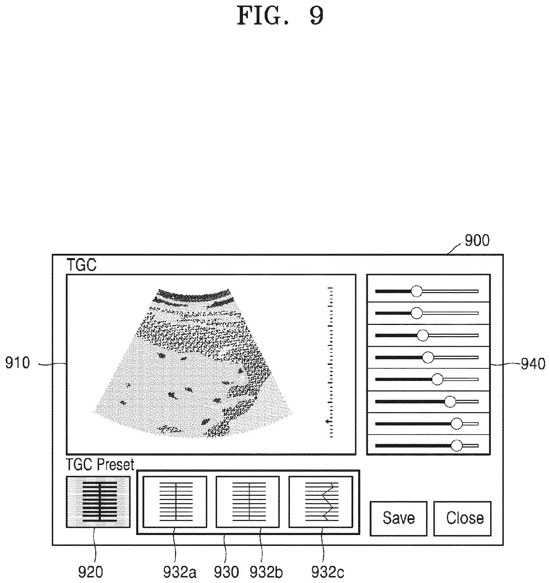

FIG. 9 illustrates a GUI view 900 for TGC control of the ultrasound imaging apparatus 310 or the client apparatus 320, according to an embodiment.

According to an embodiment, the ultrasound imaging apparatus 310 or the client apparatus 320 may provide a plurality of predefined TGC curve options 932a, 932b, and 932c and set a TGC parameter based on a user input of selecting one of the plurality of predefined TGC curve options 932a, 932b, and 932c. According to an embodiment, the GUI view 900 for TGC control may include an ultrasound image 910, currently set TGC curve information 920, a TGC curve option selection menu 930, and a TGC parameter setting unit 940. The TGC curve option selection menu 930 may include the plurality of predefined TGC curve options 932a, 932b, and 932c. A TGC parameter setting method used by the TGC parameter setting unit 940 of FIG. 9 is merely an example and may vary according to circumstances.

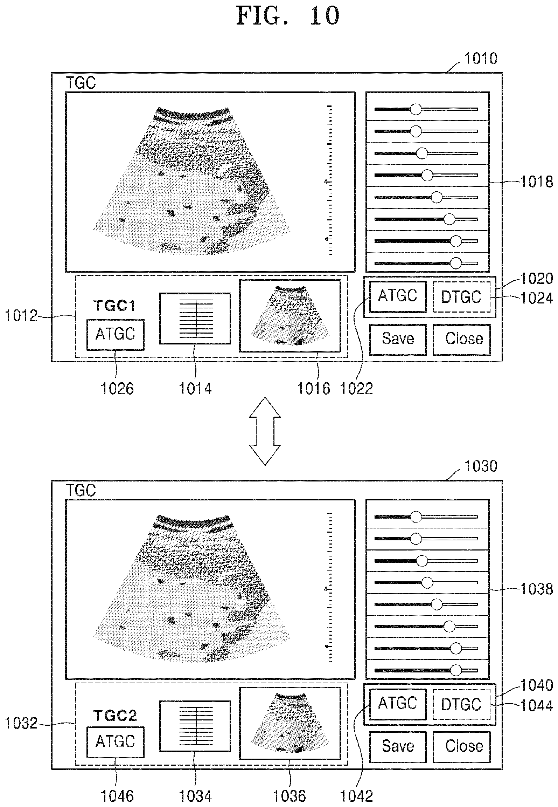

FIG. 10 illustrates GUI views 1010 and 1030 for TGC control of the client apparatus 320 and the ultrasound imaging apparatus 310, according to an embodiment.

According to an embodiment, the client apparatus 320 may provide information 1012 about a TGC setting of the ultrasound imaging apparatus 310 via the GUI view 1010. The information 1012 about the TGC setting of the ultrasound imaging apparatus 310 may include a TGC curve 1014 and a first ultrasound image 1016. Furthermore, the ultrasound imaging apparatus 310 may provide information 1032 about a TGC setting of the client apparatus 320 via the GUI view 1030. The information 1032 about the TGC setting of the client apparatus 320 may include a TGC curve 1034 and a first ultrasound image 1036.

According to an embodiment, the client apparatus 320 may provide information 1020 about a TGC method. For example, when the client apparatus 320 performs analog TGC, an icon 1022 corresponding to the analog TGC may be highlighted. On the other hand, when the client apparatus 320 performs digital TGC, an icon 1024 corresponding to the digital TGC may be highlighted. A user may select the analog TGC or digital TGC by using the corresponding icons 1022 or 1024. Furthermore, according to an embodiment, the client apparatus 320 may provide information 1026 indicating whether the TGC method performed by the ultrasound imaging apparatus 310 is analog TGC or digital TGC while providing the information 1012 about the TGC setting of the ultrasound imaging apparatus 310.

According to an embodiment, the ultrasound imaging apparatus 310 may provide information 1040 about a TGC method. For example, when the ultrasound imaging apparatus 310 performs analog TGC, an icon 1042 corresponding to the analog TGC may be highlighted. On the other hand, when the ultrasound imaging apparatus 310 performs digital TGC, an icon 1044 corresponding to the digital TGC may be highlighted. The user may select the analog TGC or digital TGC by using the corresponding icons 1042 or 1044. Furthermore, according to an embodiment, the ultrasound imaging apparatus 310 may provide information 1046 indicating whether the TGC method performed by the client apparatus 320 is analog TGC or digital TGC while providing the information 1032 about the TGC setting of the client apparatus 320.

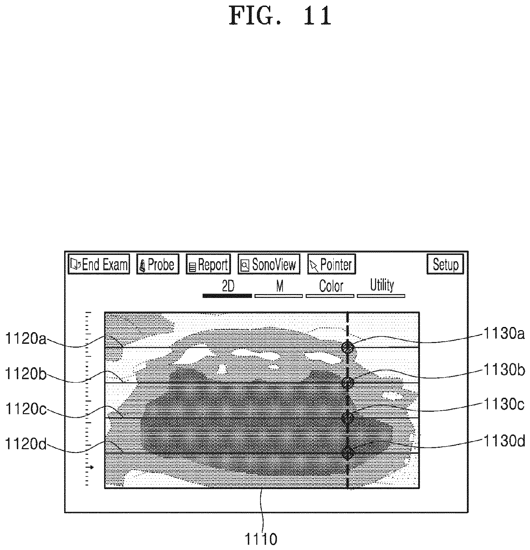

FIG. 11 illustrates a GUI view for TGC control of the ultrasound imaging apparatus 310 or the client apparatus 320, according to an embodiment.

According to an embodiment, the GUI view for TGC control may include a plurality of lines 1120a, 1120b, 1120c, and 1120d respectively corresponding to reception depths and icons 1130a, 1130b, 1130c, and 1130d respectively corresponding thereto. A user may adjust each TGC gain value corresponding to each of the reception depths by moving each of the icons 1130a, 1130b, 1130c, and 1130d corresponding to each of the reception depths to the left or right side of an ultrasound image 1110. In an embodiment, the user may decrease a TGC gain value by moving each of the icons 1130a, 1130b, 1130c, and 1130d respectively corresponding to the reception depths to the left side of the ultrasound image 1110 and increase the TGC gain value by moving each of the icons 1130a, 1130b, 1130c, and 1130d to the right side thereof. The user may move the icons 1130a, 1130b, 1130c, and 1130d via a touch screen, a mouse, a keyboard, a knob, etc.

Furthermore, according to an embodiment, the plurality of lines 1120a, 1120b, 1120c, and 1120d and the icons 1130a, 1130b, 1130c, and 1130d respectively corresponding to the reception depths may be arranged on the ultrasound image 1110. In this case, each of the plurality of lines 1120a, 1120b, 1120c, and 1120d may be located at a position on the ultrasound image 1110, which corresponds to a reception depth of the line.

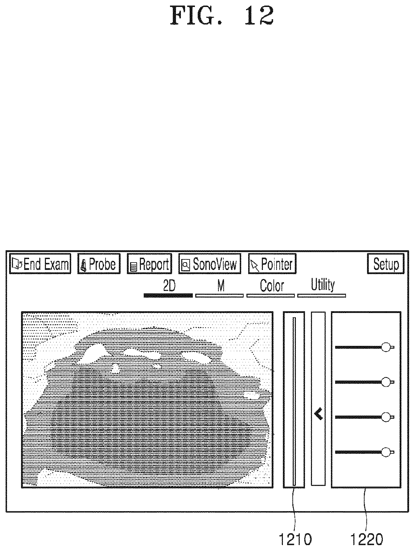

FIG. 12 illustrates a GUI view for TGC control of the ultrasound imaging apparatus 310 or the client apparatus 320, according to an embodiment.

According to an embodiment, the GUI view for TGC control may provide a TGC curve representing a currently set TGC parameter. According to an embodiment, the GUI view may include a first region 1220 composed of a plurality of lines and icons respectively corresponding to reception depths and a TGC curve region 1210 corresponding to a set TGC parameter.

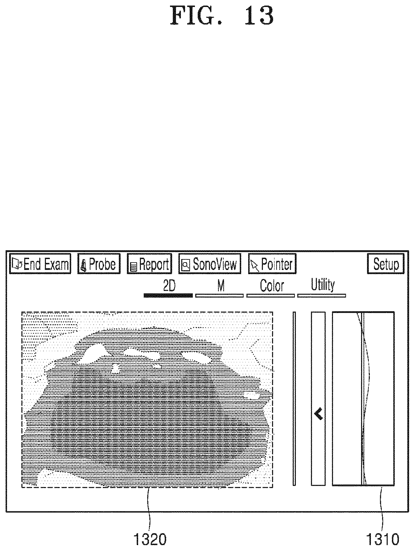

FIG. 13 illustrates a GUI view for TGC control of the ultrasound imaging apparatus 310 or the client apparatus 320, according to an embodiment.

According to an embodiment, the GUI view for TGC control may be used to receive a user input of drawing a TGC curve in a second region 1310 and display the TGC curve corresponding to the received user input in the second region 1310. According to an embodiment, an ultrasound image 1320 and the second region 1310 may be arranged parallel to each other, and a reception depth in the ultrasound image 1320 and a corresponding reception depth in the TGC curve may be shown by a horizontal line.

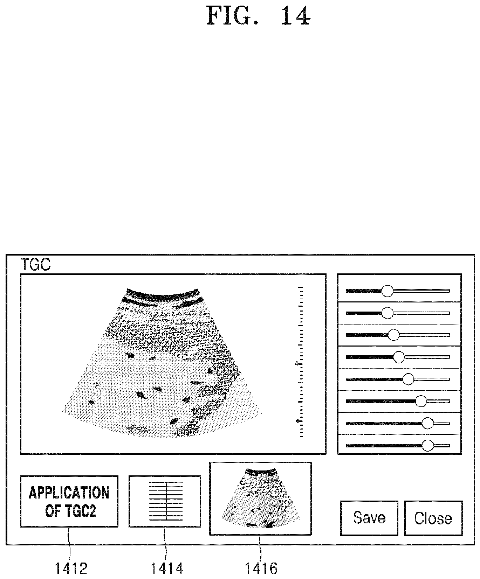

FIG. 14 illustrates a GUI view for TGC control of the ultrasound imaging apparatus 310, according to an embodiment.

According to an embodiment, which is not part of the invention, the ultrasound imaging apparatus 310 may apply a second TGC parameter for the client apparatus 320 to the ultrasound imaging apparatus 310 itself. The GUI view for TGC control of the ultrasound imaging apparatus 310, may include a first icon 1412 for selecting application of the second TGC parameter to the ultrasound imaging apparatus 310. In addition, the GUI view of the ultrasound imaging apparatus 310 may include information 1414 about the second TGC parameter and a second ultrasound image 1416. When the user selects the first icon 1412, the second TGC parameter may be applied to a first TGC channel.

According to embodiments, a client apparatus is capable of controlling a TGC parameter of an ultrasound imaging apparatus.

Furthermore, according to the embodiments, which are not part of the invention, client apparatus may adjust an analog TGC parameter and separately generate and provide an image to an ultrasound imaging apparatus and an image to a client apparatus.

Embodiments may be implemented through non-transitory computer-readable recording media having recorded thereon computer-executable instructions and data. The instructions may be stored in the form of program codes, and when executed by a processor, generate a predetermined program module to perform a specific operation. Furthermore, when being executed by the processor, the instructions may perform specific operations according to the embodiments.

## Claims

1. An ultrasound imaging apparatus (310a), the ultrasound imaging apparatus comprising:
a probe (20, 410) including a transducer array;
an ultrasound signal receiver (115, 420) including a first time gain compensation (TGC) channel (510) and a second TGC channel (520) that each perform TGC on an ultrasound signal received from the probe, wherein the first TGC channel is configured to generate a first ultrasound signal by applying a first TGC parameter to a signal received from the probe and the second TGC channel is configured to generate a second ultrasound signal by applying a second TGC parameter to a signal received from the probe;
one or more processors (430) configured to control the first TGC parameter of the first TGC channel based on a first TGC control signal received from the ultrasound imaging apparatus, generate first ultrasound image based on the first ultrasound signal received from the ultrasound signal receiver, and display the first ultrasound image on a display of the ultrasound imaging apparatus; and
a communicator (440) configured to receive a second TGC control signal from a first client apparatus (320a);
wherein the one or more processors (430) are further configured to control the second TGC parameter of the second TGC channel based on the second TGC control signal, generate second ultrasound image based on the second ultrasound signal received from the ultrasound signal receiver , and transmit the second ultrasound image to the first client apparatus via the communicator wherein the first TGC channel is separated from the second TGC channel.

2. The ultrasound imaging apparatus of claim 1, wherein the first TGC channel comprises a first TGC compensator configured to compensate for the signal received from the probe by applying a first TGC set that is a set of gain values at reception depths,
wherein the second TGC channel comprises a second TGC compensator configured to compensate for the signal received from the probe by applying a second TGC set that is a set of gain values at reception depths.

3. The ultrasound imaging apparatus of claim 1, wherein the one or more processors are further configured to generate a live streaming signal from the second ultrasound image and transmit the live streaming signal to the first client apparatus via the communicator.

4. The ultrasound imaging apparatus of claim 1, wherein the ultrasound signal receiver further comprises a third TGC channel, and
wherein the one or more processors are further configured to control a third TGC parameter of the third TGC channel, based on a third TGC control signal received from a second client apparatus, generate a third ultrasound image based on a third ultrasound signal produced via the third TGC channel, and transmit the third ultrasound image to the second client apparatus via the communicator.

5. The ultrasound imaging apparatus of claim 1, wherein the communicator is further configured to receive an information indicating selection of an analog TGC from the first client apparatus, and
wherein the one or more processors are further configured to perform the analog TGC on the second ultrasound image based on the second TGC parameter of the second TGC channel.

6. The ultrasound imaging apparatus of claim 1, wherein the communicator is further configured to receive an information indicating selection of a digital TGC from the first client apparatus, and
wherein the one or more processors are further configured to perform the digital TGC on the second ultrasound image based on the second TGC parameter of the second TGC channel.

7. The ultrasound imaging apparatus of claim 1, wherein the one or more processors are further configured to provide via the display at least one or a combination of information about the first TGC parameter of the first TGC channel, information about the second TGC parameter of the second TGC channel, and information about which TGC parameter is used to generate a displayed ultrasound image.

8. The ultrasound imaging apparatus of claim 1, further comprising a storage storing information about a TGC parameter set via a user input by a user of the first client apparatus, wherein the one or more processors are further configured to set the second TGC parameter of the second TGC channel to be the TGC parameter stored in the storage.

9. A computer implemented method of controlling an ultrasound imaging apparatus including a first time gain compensation (TGC) channel, a second TGC channel that each perform TGC on an ultrasound signal received via a probe and a communicator, the method comprising:
controlling a first TGC parameter of the first TGC channel based on a first TGC control signal received from the ultrasound imaging apparatus;
generating a first ultrasound signal by applying the first TGC parameter to a signal received from the probe (S604) ;
generating first ultrasound image based on the first ultrasound signal, and displaying the first ultrasound image on a display of the ultrasound imaging apparatus;
receiving a second TGC control signal from a first client apparatus via the communicator (S602) ;
controlling a second TGC parameter of the second TGC channel based on the second TGC control signal;
generating a second ultrasound signal by applying the second TGC parameter to a signal received from the probe;
generating the second ultrasound image based on the second ultrasound signal (S606), and transmitting the second ultrasound image to the first client apparatus via the communicator (S608)
wherein the first TGC channel is separated from the second TGC channel.

10. The method of claim 9,
wherein the generating a first ultrasound signal comprises compensating for the signal received from the probe by applying the first TGC set by using a first TGC compensator included in said first TGC channel; and
wherein the generating a second ultrasound signal comprises compensating for the signal received from the probe by applying the second TGC set by using a second TGC compensator included in said second TGC channel.

11. The method of claim 9, further comprising transmitting a live streaming signal generated from the second ultrasound image to the first client apparatus.

12. A computer program product comprising a non-transitory computer-readable recording medium having stored therein a computer program code for performing the method of claim 9.

## Patentansprüche

1. Ultraschallbildgebungsvorrichtung (310a), wobei die Ultraschallbildgebungsvorrichtung Folgendes umfasst:
eine Sonde (20, 410) mit einem Wandlerarray;
einen Ultraschallsignalempfänger (115, 420) mit einem ersten Zeitverstärkungskompensationskanal (TGC-Kanal, Time Gain Compensation) (510) und einem zweiten TGC-Kanal (520), die jeweils TGC an einem aus der Sonde empfangenen Ultraschallsignal durchführen, wobei der erste TGC-Kanal dazu konfiguriert ist, ein erstes Ultraschallsignal durch Anwenden eines ersten TGC-Parameters auf ein aus der Sonde empfangenes Signal zu erzeugen, und der zweite TGC-Kanal dazu konfiguriert ist, ein zweites Ultraschallsignal durch Anwenden eines zweiten TGC-Parameters auf ein aus der Sonde empfangenes Signal zu erzeugen;
einen oder mehrere Prozessoren (430), die dazu konfiguriert sind, den ersten TGC-Parameter des ersten TGC-Kanals basierend auf einem aus der Ultraschallbildgebungsvorrichtung empfangenen ersten TGC-Steuersignal zu steuern, ein erstes Ultraschallbild basierend auf dem aus dem Ultraschallsignalempfänger empfangenen ersten Ultraschallsignal zu erzeugen, und das erste Ultraschallbild auf einer Anzeige der Ultraschallbildgebungsvorrichtung anzuzeigen; und
einen Kommunikator (440), der dazu konfiguriert ist, ein zweites TGC-Steuersignal aus einer ersten Client-Vorrichtung (320a) zu empfangen;
wobei der eine oder die mehreren Prozessoren (430) ferner dazu konfiguriert sind, den zweiten TGC-Parameter des zweiten TGC-Kanals basierend auf dem zweiten TGC-Steuersignal zu steuern, ein zweites Ultraschallbild basierend auf dem aus dem Ultraschallsignalempfänger empfangenen zweiten Ultraschallsignal zu erzeugen, und das zweite Ultraschallbild über den Kommunikator an die erste Client-Vorrichtung zu übertragen;
wobei der erste TGC-Kanal von dem zweiten TGC-Kanal getrennt ist.

2. Ultraschallbildgebungsvorrichtung nach Anspruch 1, wobei der erste TGC-Kanal einen ersten TGC-Kompensator umfasst, der dazu konfiguriert ist, das aus der Sonde empfangene Signal durch Anwenden eines ersten TGC-Satzes zu kompensieren, bei dem es sich um einen Satz von Verstärkungswerten in Empfangstiefen handelt,
wobei der zweite TGC-Kanal einen zweiten TGC-Kompensator umfasst, der dazu konfiguriert ist, das aus der Sonde empfangene Signal durch Anwenden eines zweiten TGC-Satzes zu kompensieren, bei dem es sich um einen Satz von Verstärkungswerten in Empfangstiefen handelt.

3. Ultraschallbildgebungsvorrichtung nach Anspruch 1, wobei der eine oder die mehreren Prozessoren ferner dazu konfiguriert sind, ein Live-Streaming-Signal aus dem zweiten Ultraschallbild zu erzeugen und das Live-Streaming-Signal über den Kommunikator an die erste Client-Vorrichtung zu übertragen.

4. Ultraschallbildgebungsvorrichtung nach Anspruch 1, wobei der Ultraschallsignalempfänger ferner einen dritten TGC-Kanal umfasst, und
wobei der eine oder die mehreren Prozessoren ferner dazu konfiguriert sind, einen dritten TGC-Parameter des dritten TGC-Kanals basierend auf einem aus einer zweiten Client-Vorrichtung empfangenen dritten TGC-Steuersignal zu steuern, ein drittes Ultraschallbild basierend auf einem über den dritten TGC-Kanal produzierten dritten Ultraschallsignal zu erzeugen, und das dritte Ultraschallbild über den Kommunikator an die zweite Client-Vorrichtung zu übertragen;

5. Ultraschallbildgebungsvorrichtung nach Anspruch 1, wobei der Kommunikator ferner dazu konfiguriert ist, eine Information aus der ersten Client-Vorrichtung zu empfangen, die die Auswahl eines analogen TGC anzeigt, und
wobei der eine oder die mehreren Prozessoren ferner dazu konfiguriert sind, den analogen TGC basierend auf dem zweiten TGC-Parameter des zweiten TGC-Kanals auf dem zweiten Ultraschallbild durchzuführen.

6. Ultraschallbildgebungsvorrichtung nach Anspruch 1, wobei der Kommunikator ferner dazu konfiguriert ist, eine Information aus der ersten Client-Vorrichtung zu empfangen, die die Auswahl eines digitalen TGC anzeigt, und
wobei der eine oder die mehreren Prozessoren ferner dazu konfiguriert sind, den digitalen TGC basierend auf dem zweiten TGC-Parameter des zweiten TGC-Kanals auf dem zweiten Ultraschallbild durchzuführen.

7. Ultraschallbildgebungsvorrichtung nach Anspruch 1, wobei der eine oder die mehreren Prozessoren ferner dazu konfiguriert sind, über die Anzeige mindestens eines oder eine Kombination der folgenden Informationen bereitzustellen: Informationen über den ersten TGC-Parameter des ersten TGC-Kanals, Informationen über den zweiten TGC-Parameter des zweiten TGC-Kanals und Informationen darüber, welcher TGC-Parameter verwendet wird, um ein angezeigtes Ultraschallbild zu erzeugen.

8. Ultraschallbildgebungsvorrichtung nach Anspruch 1, die ferner einen Speicher umfasst, der Informationen über einen durch einen Benutzer der ersten Client-Vorrichtung über eine Benutzereingabe eingestellten TGC-Parametersatz speichert,
wobei der eine oder die mehreren Prozessoren ferner dazu konfiguriert sind, den zweiten TGC-Parameter des zweiten TGC-Kanals so einzustellen, dass er der in dem Speicher gespeicherte TGC-Parameter ist.

9. Computerimplementiertes Verfahren zum Steuern einer Ultraschallbildgebungsvorrichtung mit einem ersten Zeitverstärkungskompensationskanal (TGC-Kanal, Time Gain Compensation) und einem zweiten TGC-Kanal, die jeweils TGC an einem über eine Sonde empfangenen Ultraschallsignal durchführen, und mit einem Kommunikator, wobei das Verfahren Folgendes umfasst:
Steuern eines ersten TGC-Parameters des ersten TGC-Kanals basierend auf einem aus der Ultraschallbildgebungsvorrichtung empfangenen ersten TGC-Steuersignal;
Erzeugen eines ersten Ultraschallsignals durch Anwenden des ersten TGC-Parameters auf ein aus der Sonde empfangenes Signal (S604);
Erzeugen eines ersten Ultraschallbildes basierend auf dem ersten Ultraschallsignal und Anzeigen des ersten Ultraschallbildes auf einer Anzeige der Ultraschallbildgebungsvorrichtung;
Empfangen eines zweiten TGC-Steuersignals aus einer ersten Client-Vorrichtung über den Kommunikator (S602);
Steuern eines zweiten TGC-Parameters des zweiten TGC-Kanals basierend auf dem zweiten TGC-Steuersignal;
Erzeugen eines zweiten Ultraschallsignals durch Anwenden des zweiten TGC-Parameters auf ein aus der Sonde empfangenes Signal;
Erzeugen des zweiten Ultraschallbildes basierend auf dem zweiten Ultraschallsignal (S606); und Übertragen des zweiten Ultraschallbilds an die erste Client-Vorrichtung über den Kommunikator (S608),
wobei der erste TGC-Kanal von dem zweiten TGC-Kanal getrennt ist.

10. Verfahren nach Anspruch 9,
wobei das Erzeugen eines ersten Ultraschallsignals das Kompensieren des aus der Sonde empfangenen Signals durch Anwenden des ersten TGC-Satzes unter Verwendung eines in dem ersten TGC-Kanal enthaltenen ersten TGC-Kompensators umfasst; und
wobei das Erzeugen eines zweiten Ultraschallsignals das Kompensieren des aus der Sonde empfangenen Signals durch Anwenden des zweiten TGC-Satzes unter Verwendung eines in dem zweiten TGC-Kanal enthaltenen zweiten TGC-Kompensators umfasst.

11. Verfahren nach Anspruch 9, ferner umfassend das Übertragen eines aus dem zweiten Ultraschallbild erzeugten Live-Streaming-Signals an die erste Client-Vorrichtung.

12. Computerprogrammprodukt mit einem nicht flüchtigen, computerlesbaren Aufzeichnungsmedium, auf dem ein Computerprogrammcode zum Durchführen des Verfahrens nach Anspruch 9 gespeichert ist.

## Revendications

1. Appareil échographique (310a), l'appareil échographique comprenant :
une sonde (20, 410) comportant un réseau d'éléments transducteurs ;
un récepteur de signaux ultrasonores (115, 420) comportant un premier canal de compensation du gain en fonction du temps (TGC, time gain compensation) (510) et un deuxième canal de TGC (520) qui effectuent chacun une TGC sur un signal ultrasonore reçu en provenance de la sonde, ledit premier canal de TGC étant conçu pour générer un premier signal ultrasonore par application d'un premier paramètre TGC sur un signal reçu en provenance de la sonde et ledit deuxième canal de TGC étant conçu pour générer un deuxième signal ultrasonore par application d'un deuxième paramètre TGC sur un signal reçu en provenance de la sonde ;
un ou plusieurs processeurs (430) conçus pour commander le premier paramètre TGC du premier canal de TGC compte tenu d'un premier signal de commande TGC reçu en provenance de l'appareil échographique, générer une première image échographique compte tenu du premier signal ultrasonore reçu en provenance du récepteur de signaux ultrasonores, et afficher la première image échographique sur un écran de l'appareil échographique ; et
un dispositif de communication (440) conçu pour recevoir un deuxième signal de commande TGC en provenance d'un premier appareil client (320a) ;
lesdits un ou plusieurs processeurs (430) étant conçus en outre pour commander le deuxième paramètre TGC du deuxième canal de TGC compte tenu du deuxième signal de commande TGC, générer une deuxième image échographique compte tenu du deuxième signal ultrasonore reçu en provenance du récepteur de signaux ultrasonores, et transmettre la deuxième image échographique au premier appareil client par le biais du dispositif de communication ;
ledit premier canal de TGC étant séparé du deuxième canal de TGC.

2. Appareil échographique selon la revendication 1, dans lequel le premier canal de TGC comprend un premier compensateur TGC conçu pour compenser le signal reçu en provenance de la sonde par application d'un premier ensemble de TGC, consistant en un ensemble de valeurs de gain aux profondeurs de réception,
dans lequel le deuxième canal de TGC comprend un deuxième compensateur TGC conçu pour compenser le signal reçu en provenance de la sonde par application d'un deuxième ensemble de TGC, consistant en un ensemble de valeurs de gain aux profondeurs de réception.

3. Appareil échographique selon la revendication 1, dans lequel les un ou plusieurs processeurs sont conçus en outre pour générer un signal de diffusion continue en direct à partir de la deuxième image échographique et transmettre le signal de diffusion continue en direct au premier appareil client par le biais du dispositif de communication.

4. Appareil échographique selon la revendication 1, dans lequel le récepteur de signaux ultrasonores comprend en outre un troisième canal de TGC, et
dans lequel les un ou plusieurs processeurs sont conçus en outre pour commander un troisième paramètre TGC du troisième canal de TGC, compte tenu d'un troisième signal de commande TGC reçu en provenance d'un deuxième appareil client, générer une troisième image échographique compte tenu d'un troisième signal ultrasonore produit par le biais du troisième canal de TGC, et transmettre la troisième image échographique au deuxième appareil client par le biais du dispositif de communication.

5. Appareil échographique selon la revendication 1, dans lequel le dispositif de communication est conçu en outre pour recevoir une information indiquant la sélection d'une TGC analogique en provenance du premier appareil client, et
dans lequel les un ou plusieurs processeurs sont conçus en outre pour effectuer la TGC analogique sur la deuxième image échographique compte tenu du deuxième paramètre TGC du deuxième canal de TGC.

6. Appareil échographique selon la revendication 1, dans lequel le dispositif de communication est conçu en outre pour recevoir une information indiquant la sélection d'une TGC numérique en provenance du premier appareil client, et
dans lequel les un ou plusieurs processeurs sont conçus en outre pour effectuer la TGC numérique sur la deuxième image échographique compte tenu du deuxième paramètre TGC du deuxième canal de TGC.

7. Appareil échographique selon la revendication 1, dans lequel les un ou plusieurs processeurs sont conçus en outre pour fournir, par le biais de l'écran, au moins une information ou une combinaison d'informations concernant le premier paramètre TGC du premier canal de TGC, concernant le deuxième paramètre TGC du deuxième canal de TGC et concernant celui des paramètres TGC utilisé pour générer une image échographique affichée.

8. Appareil échographique selon la revendication 1, comprenant en outre un dispositif de stockage qui stocke des informations concernant un paramètre TGC réglé par le biais d'une entrée effectuée par l'utilisateur du premier appareil client, et
dans lequel les un ou plusieurs processeurs sont conçus en outre pour régler, comme paramètre TGC stocké dans le dispositif de stockage, le deuxième paramètre TGC du deuxième canal de TGC.

9. Procédé mis en œuvre par voie informatique pour commander un appareil échographique comportant un premier canal de compensation du gain en fonction du temps (TGC, time gain compensation) et un deuxième canal de TGC qui effectuent chacun une TGC sur un signal ultrasonore reçu par le biais d'une sonde, et un dispositif de communication, le procédé comprenant :
la commande d'un premier paramètre TGC du premier canal de TGC compte tenu d'un premier signal de commande TGC reçu en provenance de l'appareil échographique,
la génération d'un premier signal ultrasonore par application du premier paramètre TGC sur un signal reçu en provenance de la sonde (S604),
la génération d'une première image échographique compte tenu du premier signal ultrasonore, et l'affichage de la première image échographique sur un écran de l'appareil échographique,
la réception d'un deuxième signal de commande TGC en provenance d'un premier appareil client par le biais du dispositif de communication (S602),
la commande d'un deuxième paramètre TGC du deuxième canal de TGC compte tenu du deuxième signal de commande TGC,
la génération d'un deuxième signal ultrasonore par application du deuxième paramètre TGC sur un signal reçu en provenance de la sonde,
la génération de la deuxième image échographique compte tenu du deuxième signal ultrasonore (S606), et
la transmission de la deuxième image échographique au premier appareil client par le biais du dispositif de communication (S608) ;
ledit premier canal de TGC étant séparé du deuxième canal de TGC.

10. Procédé selon la revendication 9,
dans lequel la génération d'un premier signal ultrasonore comprend la compensation du signal reçu en provenance de la sonde par application du premier ensemble de TGC au moyen d'un premier compensateur TGC inclus dans ledit premier canal de TGC, et
dans lequel la génération d'un deuxième signal ultrasonore comprend la compensation du signal reçu en provenance de la sonde par application du deuxième ensemble de TGC au moyen d'un deuxième compensateur TGC inclus dans ledit deuxième canal de TGC.

11. Procédé selon la revendication 9, comprenant en outre la transmission d'un signal de diffusion continue en direct, généré à partir de la deuxième image échographique, au premier appareil client.

12. Produit-programme d'ordinateur comprenant un support d'enregistrement non transitoire lisible par ordinateur sur lequel est stocké un code de programme informatique permettant de réaliser le procédé selon la revendication 9.
